# EUROPEAN PATENT APPLICATION

(11) **EP 1 980 625 A1**
(43) Date of publication of application: **15.10.2008**
(21) Application number: 07105879.6
(22) Date of filing: 10.04.2007
(51) Int. Cl.: C12Q 1/68

(54) **Means and methods for haplotyping MHC-DRB loci in mammals and uses thereof**

(71) Applicant: Stichting Biomedical Primate Research Centre, 2288 GJ Rijswijk (NL)
(72) Inventor: Bontrop, Ronald Edward, 2253 JL Voorschoten (NL); Doxiadis, Gabriele Gerda Maria, 2314 AK Leiden (NL)
(74) Representative: van Loon, C.J.J.

(57) **Abstract**

The invention relates to the typing of MHC-DRB loci in mammals. In particular, the invention provides a typing procedure for the mammalian DRB region that allows an easy, economical, high resolution, fast and accurate haplotyping protocol. The invention further provides the use of said typing procedure in genetic applications, and provides a kit for typing of MHC-DRB loci.

## Description

The invention relates to the field of biology and medicine. In particular, the invention relates to the typing of MHC-DRB loci in mammals.

The Major Histocompatibility Complex (MHC) is a large genomic region that is present in most vertebrates, although individual MHC region-linked genes have been identified in invertebrate genomes such as *Drosophila melanogaster* and *Caenorhabditis elegans.* The MHC gene products play a key role in adaptive immunology. Genes of the MHC are among the most polymorphic loci known in vertebrates, due to a high rate of mutation and recombination. Major differences between species reside not only in the number and identity of MHC genes, but also in the degree of allelic polymorphism for individual genes.

In general, three subgroups of MHC molecules can be defined, termed class I, II and III gene products. Molecules encoded by the so-called classical MHC class I and class II genes, present short peptides derived from proteins of pathogens and self antigens to the immune system. MHC class I genes encode molecules that are present on the surfaces of nearly all nucleated cells. These gene products are mainly responsible for facilitating immune responses to intracellularly processed pathogens such as viruses. MHC class II gene products are mainly present in a subset of antigen-presenting cells such as B-lymphocytes and activated T-lymphocytes. Class II gene products are primarily involved in the immune response against extracellular pathogens such as bacteria. Furthermore, they play an important role in recognition of foreign and self-antigens. MHC class III gene products are not involved in antigen presentation but rather represent other immune-related components such as components of the complement system.

Of particular importance are the MHC genes that encode the cell surface, antigen-presenting molecules. In humans, these genes are referred to as Human Leukocyte Antigen (HLA) genes. The classical MHC genes HLA-A, HLA-B, and HLA-C belong to MHC class I, encoding the alpha chain of the respective MHC molecule. The HLA class II region is divided into -DP, -DQ, and -DR. The classical HLA-DR, -DQ, and -DP molecules are transmembrane heterodimers, composed of an alpha- and beta-chain subunit encoded by the A and B genes, respectively.

Defects in some MHC class II genes have been associated with autoimmune disorders such as arthritis and diabetes (Otsuka et al.. 2006. Proc Natl Acad Sci U S A. 103: 14465-7; Aoki et al. 2005. Autoimmun Rev. 4: 373-9). Similarly, polymorphisms in MHC class II genes have been associated with susceptibility to a range of infectious diseases including malaria, tuberculosis, leprosy, typhoid fever, hepatitis and HIV/AIDS. For instance, experimental autoimmune encephalomyelitis in rhesus macaques, a model for the human disease multiple sclerosis, is known to be influenced by certain MHC class II alleles (Slierendrecht et al. 1995. International Immunology, Vol. 7: 1671-1679).

In the human population, five major -DRB region configurations are classified. These region configurations share an invariant HLA-DRA gene and a -DRB9 gene segment but differ in physical length and also in the composition and number of other DRB loci. Like humans, other primates such as chimpanzees, gorillas, and rhesus macaques have variable numbers of MHC-DRB loci per haplotype (Doxiadis et al. 2000. J. of immunol. 164: 3193-3199). For example, the DRB region configuration in rhesus macaques (Macaca mulatta), termed Mamu-DRB, is highly plastic and has been subject to various contractions and expansions (Slierendregt et al. 1994. J Immunol 152: 2298-307). In humans a high degree of polymorphism is observed for the DRB1 locus present on all haplotypes (Robinson et al. 2003. Nucleic Acids Res 31: 311-4). In rhesus macaques, however, a high degree of region configuration polymorphism has been described characterized by marked differences with regard to number and content of distinct loci present per haplotype (Doxiadis et al. 2000. J Immunol 164: 3193-9; Doxiadis et al. 2001. Immunol Rev 183: 76-85). The Mamu-DRB region configurations themselves, however, display a relatively low degree of polymorphism. Most of the Mamu-DRB alleles belong to loci/lineages that are shared with humans (Bontrop et al. 1999. Immunol Rev 167: 339-50), and their alleles have been named accordingly (Klein et al. 1990. Immunogenetics 31: 217-9). Similar observations regarding sharing of certain MHC-DRB loci/lineages with human orthologues have been made for other primate species, whereas DRB alleles are mostly species specific (Bontrop et al. 1999. Immunol Rev 167: 339-50).

It is generally accepted that allelic polymorphisms of the MHC class II genes warrant that different allotypes select distinct peptides for T cell activation, preventing one particular pathogen from sweeping through an entire population. Most sequence variability is confined to exon 2 of the MHC-DPB, -DQA, -DQB, and -DRB genes. One of the most polymorphic regions in humans is the HLA-DRB region with more than 500 alleles described worldwide until now (Bodmer et al. 1999. Eur J. Immunogenet. 26: 81; see also http://www.ebi.ac.uk/imgt/hla/stats.html). In rhesus macaques, a species far less analysed than humans, already more than 135 Mamu-DRB distinct genes/alleles have been determined, a number which will increase rapidly when more animals are analysed.

Due to the complexity of the DRB-region, the existing typing procedures, involving single-strand conformation polymorphism, denaturing gradient gel electrophoresis (DGGE), restriction fragment length polymorphism analyses, or sequence analyses, are cumbersome and time consuming. It is therefore an object of the present invention to provide a typing procedure for the mammalian DRB region that allows an easy, economical, high resolution, fast and accurate haplotyping protocol.

Therefore, in one aspect the present invention provides a method for determining a DRB haplotype in a sample comprising nucleic acid from a mammalian cell, said method comprising amplifying at least an intron 2 microsatellite-containing part of at least one DRB-gene from said nucleic acid; determining the type of intron 2 microsatellite present in said at least one DRB-gene; and determining the DRB haplotype of said cell from said type of intron 2 microsatellite present in said at least one DRB-gene.

The inventors have established that the type of intron 2 microsatellite present in said at least one DRB-gene is indicative of the DRB-allele that is associated with said intron in a cis-configuration, thus allowing the identification of said DRB-allele by typing of the associated intron 2 microsatellite. An advantage of said method is that the typing of said intron 2 microsatellite can be performed by relatively simple methods which do not involve complicated methods such as single-strand conformation polymorphism or sequence analyses. Therefore, said method provides an easy and economical method for determining a DRB haplotype in a sample.

A complex dinucleotide repeat or microsatellite, D6S2878, is located in the beginning of intron 2 of the DRB genes of human and non-human primates (Bergstroem et al. 1999. Am J Hum Genet 64: 1709-1718; Epplen et al. 1997. Hum Genet 99: 399-406; Riess et al. 1990. Immunogenetics 32:110-116). This microsatellite displays a complex polymorphism profile concerning repeat length variability as well as sequence variation. According to the present invention, the variability of said microsatellite can be used for typing of the cis-associated DRB-allele. The number of and identity of distinct DRB-alleles that are present in a sample, as determined by the number and types of intron 2 microsatellites that are present in said sample, is used to determine a DRB-haplotype.

The number and types of intron 2 microsatellites that are present in a sample can be determined by any method known in the art, including but not limited to sequence analyses and sequence-specific amplification. However, a quick and convenient method for determining the number and types of intron 2 microsatellites comprises the determination of the length of the microsatellite, as it was found that the length of the intron 2 microsatellite is indicative for the cis-associated DRB-allele.

Therefore, in a preferred embodiment the invention provides a method for determining a DRB haplotype, wherein the typing of the intron 2 microsatellite is based on the length of said microsatellite.

Thus, a DRB-haplotype can be determined by analysis of the length of intron 2 microsatellite that is present in the amplified fragments from the at least one DRB gene. Each haplotype is identified by a specific number and size of the amplified fragments. Thus by identifying the specific number and size of the amplified fragments, the haplotype can be identified.

The number of distinct fragments obtained after amplification will depend on the number of distinct DRB-alleles that are present in a particular DRB-haplotype. The total number of DRB-alleles that can be present in a sample, and consequently resulting in multiple distinct amplified fragments, also depends on whether the cell is homozygous or heterozygous for a DRB-haplotype, as in the latter case more distinct fragments might be present.

In a preferred embodiment, the type of DRB-allele is determined by comparing the amplified intron 2 microsatellite-derived fragments with a reference. Said reference can be a sample from a individual of which the types of DRB-alleles and the corresponding DRB-haplotype have been previously determined. Said reference can, for example, be taken along in the amplification reaction. The reference sample can be taken from the same or a different species, however, it is preferred that said reference sample is taken from the same species.

Therefore, the invention also provides a method wherein a DRB haplotype is determined by comparing the detected types of intron 2 microsatellite with a reference.

In preferred embodiment, said reference is taken from a sample from an individual of which the types of DRB-genes and the corresponding DRB-haplotype have been previously determined and of which relevant data, comprising number and length and/or sequence of the amplified intron 2 microsatellite fragments, have been stored in a database.

Therefore, in this embodiment, the invention provides a method wherein a type of intron 2 microsatellite is determined by comparing with a reference comprising a database of DRB haplotypes correlated with the associated intron 2 microsatellite types.

In a preferred embodiment, a database according to the invention is present in an electronic storage device, such as, but not limited to, a computer or a server. It is further preferred that said database comprising said reference can be addressed to compare the amplified intron 2 microsatellite-derived fragments with said reference.

Amplification of at least intron 2, or a microsatellite-containing portion thereof, of a DRB-gene can be performed by any method known in the art including, but not limited to, polymerase chain reaction, strand displacement amplification, nucleic acid sequence-based amplification, rolling circle amplification technology, and transcription-mediated amplification. Each of these amplification methods uses different approaches to achieve the amplification of nucleic acid molecules to amounts that can subsequently be detected.

In a preferred embodiment, the invention also provides a primer pair that spans the region containing said intron 2 microsatellite, for amplifying at least said intron 2 microsatellite-containing part of at least one DRB-gene.

According to this embodiment, said primer pair is preferably selected to allow amplification of at least 1 DRB gene, more preferred at least 2 DRB genes, more preferred at least 3 DRB genes, more preferred at least 4 DRB genes, more preferred at least 5 DRB genes, more preferred at least 6 DRB genes, more preferred at least 7 DRB genes, more preferred at least 10 DRB genes, that are present within the DRB region of one or more individuals of a species.

Therefore, each of the primers of said primer pair preferably is selected to hybridize to a nucleic acid region that is conserved in most or essentially all DRB-genes and alleles thereof within a species, thereby allowing the amplification of said intron 2 microsatellite of essentially all DRB genes and alleles thereof that are present within the DRB region of one or more individuals of a species.

In a preferred embodiment, said primer pair comprises a first primer of which the nucleotide sequences corresponds to a conserved nucleotide sequence that is present in a position that is adjacent to the microsatellite in intron 2, and a second primer of which the nucleotide sequences corresponds to a conserved sequence that is present on the opposite side of said microsatellite sequence, relative to the first primer.

In a preferred embodiment, said first primer is selected from a region spanning the 3' end of exon 2 and the 5'start of intron 2 of a DRB gene. This region was found to be conserved within species, but not between species. A second primer can be selected from a region comprising a conserved sequence, said region being present within a range of up to 1000 nucleotides from the microsatellite at the opposite side of the microsatellite in intron 2 of a DRB gene relative to said first primer.

In a more preferred embodiment, said second primer is selected from a region comprising conserved sequences that are present within a range of 5 to 60 nucleotides from the microsatellite at the opposite side of the microsatellite in intron 2 of a DRB gene, relative to said one primer.

Therefore, in an even further preferred embodiment, a primer pair according to the invention comprises a first primer of which the nucleotide sequences corresponds to a conserved region spanning the 3' end of exon 2 and the 5' start of intron 2 of a DRB gene, and a second primer of which the nucleotide sequences corresponds to a region comprising conserved sequences that is present within a range of 5 to 60 nucleotides from the microsatellite at the opposite side of the microsatellite in intron 2 of a DRB gene, relative to said first primer.

In yet an even further preferred embodiment, said first primer is chosen from the primers
GAGAGCTTCACAGTGCAGCG (SEQ ID NO 1);
TTCACAGTGCAGCGGCGAGGT (SEQ ID NO 2); and
CGTGTCCCCACAGCACGTTTC (SEQ ID NO 6)
and said second primer is chosen from the primers
GAGAGGATTCTGAATGCTCA (SEQ ID NO 3);
ACACCTGTGCCCTCAGAACT (SEQ ID NO 4); and
ACATCTGTGTCCTCAGACCT (SEQ ID NO 5).

It is furthermore preferred that the amplified nucleic acid molecules comprising at least intron 2 of a DRB gene, or a microsatellite-containing portion thereof that are derived from different DRB-alleles can be discriminated by physical characteristics such as size, isoelectric point, or nucleotide sequence of the molecules. Therefore, the length of the amplified fragment is preferably less than 1000 nucleotides, more preferred less than 900 nucleotides, more preferred less than 800 nucleotides, more preferred less than 700 nucleotides, more preferred less than 600 nucleotides, more preferred less than 500 nucleotides, more preferred less than 400 nucleotides, more preferred less than 300 nucleotides.

An amplified fragment comprising the microsatellite can be detected by any method known in the art. Detection methods include real-time detection methods such as DNA binding fluorophores, 5' endonuclease, adjacent linear and hairpin oligoprobes, and self-fluorescing amplicons.

In another embodiment, detection of the amplified nucleic acid is performed by post-amplification methods, including but not limited to, colorimetric detection, chemiluminescence, and gel electrophoresis detection.

Gel electrophoresis comprises the analysis of nucleic acid molecules by using a polymer such as agarose or polyacrylamide. Known methods include slab gel electrophoresis and capillary gel electrophoresis. Nucleic acid molecules are separated on the polymer based on physical characteristics such as size, shape, or isoelectric point, of the molecules. Visualization of nucleic acid molecules after electrophoreses comprises classic staining procedures, including but not limited to ethidium bromide, silver, SYBR Green (2-[N-(3-dimethylaminopropyl)-N-propylamino]-4-[2,3-dihydro-3-methyl-(benzo-1,3-thiazol-2-yl)-methylidene]-1-phenyl-quinolinium; Invitrogen), coomassie blue dyes, or inorganic microparticle labels such as nanocrystals or quantum dots.

The length of an amplified nucleic acid can be determined by methods known to a skilled person, including but not limited to comparison to a known standard provided by fragments of known length, measuring of the time that is required to reach a defined position on the gel under standard conditions, and by any other method that determines the size, shape, or isoelectric point of the nucleic acid, such as mass spectrometry.

Sequence analyses of each of the amplified nucleic acid molecules, or of a suitable part thereof, can be performed using methods that are known in the art, including but not limited to, chemical analyses, enzymatic analyses using nucleotide analogues, and hybridization methods.

In a preferred embodiment, amplified nucleic acid molecules according to the invention are generated using labelled nucleotide analogues in the amplification reaction, allowing detection of said amplified nucleic acid molecules after gel electrophoresis without staining and washing procedures. Labelled nucleotide analogues that can be used include, but are not limited to, fluorescently-labelled nucleotide analogues such as cyanine-dyes (Cy3 or Cy5) -labelled analogues, and radioactively labelled nucleotide analogues.

In an alternative embodiment, amplified nucleic acid molecules are detected through the native fluorescence of nucleic acid molecules.

In a more preferred embodiment, the nucleic acid molecules are fluorescently labelled. They can be detected using any method known in the art, such as, for example, laser-induced fluorescence. Detection is based on the excitation and emission spectra of the fluorescent label that is used.

In an even more preferred embodiment, the amplified nucleic acid molecules are generated using at least one primer that is labelled. Said at least one primer can be labelled with, for example, luminescent method system such as lanthanide ions, or fluorescence-based dyes such as 6-FAM, HEX, NED, ROX, 5-FAM, JOE, Cy3, and Cy5. The use of at least one primer that is labelled will also allow detection of the amplified nucleic acid molecules by gel electrophoresis without staining and washing procedures.

Therefore, a preferred method according to the invention comprises amplifying at least an intron 2 microsatellite-containing part from a DRB gene with a primer pair, wherein one or more of the primers is labelled.

Said sample comprising nucleic acid molecules of the cell that is used for amplifying at least intron 2, or a microsatellite-containing portion thereof, may comprise any nucleic acid molecule that comprises intron 2, or at least a microsatellite-containing portion thereof. These nucleic acid molecules include, but are not limited to, unspliced RNA, such as, for example, nuclear RNA, and genomic DNA.

In a preferred embodiment, the invention provides a method for determining a DRB haplotype in a sample comprising genomic DNA.

The sample can be derived from any mammal that comprises a microsatellite in intron 2 of a DRB gene. The present invention is suitable for designing breeding programs such as used in animal husbandry and for endangered species such as whales and elephants. Non-limiting examples of a mammal comprise pets such as dog and cat; ungulates including sheep, goat, and cattle such as cow; horse; and primates.

In a preferred embodiment, the sample is derived from any human or non-human primate that comprises a microsatellite in intron 2 of a DRB gene. Not limiting examples of human or non-human primates include apes such as chimpanzee, gorilla, gibbon, siamang, orangutan, and human, Old World monkeys such as mandrill, macaque and baboon, New World monkeys , and Prosimians.

In a further preferred embodiment, a method according to the invention provides determining a DRB-haplotype in a sample that is derived from a human.

In another aspect, the invention relates to a kit for determining a DRB haplotype in a cell, said kit comprising means for amplifying at least an intron 2 microsatellite-containing part of at least one DRB-gene.

In one embodiment, said kit comprises a pair of primers that span a region of a DRB gene flanking the intron 2 microsatellite-containing part of at least one DRB-gene.

In a preferred embodiment, a first primer of said primer pair comprises sequences selected from a conserved region spanning the 3' end of exon 2 and the 5'start of intron 2 of a DRB gene, while a second primer comprises sequences selected from a conserved region that is present on the opposite side of said microsatellite sequence, relative to the first primer.

In a further preferred embodiment, said first and second primers are selected such that the length of the amplified fragment from the intron-2 of a DRB-gene is less than 1000 nucleotides, more preferred less than 900 nucleotides, more preferred less than 800 nucleotides, more preferred less than 700 nucleotides, more preferred less than 600 nucleotides, more preferred less than 500 nucleotides, more preferred less than 400 nucleotides, more preferred less than 300 nucleotides.

In another embodiment, the kit comprises primers of which a 5' primer comprises the nucleotide sequence of SEQ ID NO 1; and a 3' primer comprises the nucleotide sequence of SEQ ID NO 3.

In yet another embodiment, the kit comprises primers of which a 5' primer comprises the nucleotide sequence of SEQ ID NO 2; and a 3' primer comprises the nucleotide sequence of SEQ ID NO 4 or SEQ ID NO 5.

In a further embodiment, the kit comprises primers of which a 5' primer comprises the nucleotide sequence of SEQ ID NO 6; and a 3' primer comprises the nucleotide sequence of SEQ ID NO 3, SEQ ID NO 4 or SEQ ID NO 5.

In another aspect, the invention relates to the use of a method for determining a DRB haplotype in a sample for a genetic application.

The genes of the MHC are known the be among the most polymorphic loci known in human or non-human primates, due to a high rate of mutation and recombination. Major differences between species reside not only in the number and identity of MHC genes, but also in the degree of allelic polymorphism for individual genes. Therefore, the method for determining a DRB-haplotype in a sample, as provided by the current invention, can be used in genetic tests. Genetic test comprise tests to look for a possible predisposition to disease before any symptoms appear.
The most widespread type of genetic testing is newborn screening. Genetic test can be helpful in several areas: early detection, diagnosis, prognosis, and treatment.

In a further embodiment, the method for determining a DRB-haplotype in a sample according to the present invention can be used a genetic application comprising paternity testing.

Paternity tests can be performed postnatally by, for example, testing of blood or testing of a buccal swab, or testing of the umbilical cord, or testing of other sample collection including but not limited to semen, tissue and hair. Paternity tests can also be performed pre-natally by testing of a sample obtained by amniocentesis or by chorionic villus sampling.

In yet a further embodiment, the method for determining a DRB-haplotype in a sample according to the present invention can be used in a genetic application comprising forensic testing.

Forensic testing includes the application of method for comparison of biological samples in criminal investigations. Typically, the samples are processed in a dedicated forensic laboratory. Short tandem repeats such as provided by the microsatellite in the intron 2 of a DRB-gene may be included in a standard battery of core loci. This may increase the discriminatory power and decrease the probability of a match between profiles of two unrelated persons.

In another embodiment, the method for determining a DRB-haplotype in a sample according to the present invention can be used in a genetic application comprising tissue typing for transplantation testing.

MHC molecules play a crucial role in T-cell activation by antigen presenting cells. Antigenic recognition depends on the interaction between the antigenic peptide-binding MHC molecule and the T-Cell Receptor (TCR), an immunoglobulin-like heterodimeric protein expressed on T-lymphocytes. The transplanted organ represents a continuous source of antigens that can induce a rejection response at any time post-transplant. Furthermore, donor-derived immuno-competent lymphocytes may react with MHC-incompatible recipient cells and induce inflammatory responses in host tissues such as the skin and gastrointestinal tract. This complication is frequent after bone marrow transplantation, but may also affect recipients of liver and other organ transplants and even blood transfusions.

Therefore, transplants with well-matched antigens, especially matching of DRB-haplotype, may function significantly better than those with a poor match, due to different rates of initial immune reactions, graft rejection, and graft failure due to infection or other causes.
In yet another embodiment, the method for determining a DRB-haplotype in a sample according to the present invention can be used in a genetic application comprising testing for chronic or infectious diseases.

Determining a DRB-haplotype may help to determine a possible predisposition to a disease before any symptoms appear. These diseases include, but are not limited to, autoimmune disorders such as arthritis, diabetes, multiple sclerosis (Gregersen et al., 2006. Nature 443: 574-7), bowel diseases such as ulcerative colitis and Crohn's disease, psoriasis, and chronic fatigue syndrome. Furthermore, polymorphisms in MHC class II genes such as DRB genes, have been associated with susceptibility to a range of infectious diseases including malaria, tuberculosis, leprosy, typhoid fever, hepatitis and HIV/AIDS.

In yet another aspect, the invention relates to the use of a kit for determining a DRB haplotype in a cell, said kit comprising means for amplifying at least an intron 2 microsatellite-containing part of at least one DRB-gene, in a genetic application comprising paternity testing, forensic testing, tissue typing for transplantation testing, or testing for chronic or infectious diseases.

### Definitions

The term DRB-gene is defined as a functional unit whose inheritance can be followed experimentally. The term DRB gene comprises both an active gene and a pseudogene, the latter being defined as a sequence that closely resembles a known DRB-gene but which is not expressed.

The term DRB-allele is defined as an alternative form of a DRB-gene that can be distinguished from other forms of said DRB-gene by, for example, sequence analyses.

The term DRB-locus is defined as a position on a chromosome that corresponds to a DRB-gene.

The term DRB-haplotype is defined as the unique combination of one or more DRB genes or their evolutionary equivalents that are in most mammals associated in a cis-configuration to the MHC-DRA gene or its evolutionary equivalent on the same chromosome. A DRB-haplotype is determined by the number of DRB-genes, and the specific DRB-alleles that are present of each of the DRB-genes, within the DRB region.

### Brief description of drawings

### Figure 1

Phylogenetic analysis of HLA-DRB exon 2 with D6S2878 sequences superimposed. The tree was rooted by including the Caja-DRB*W1601 allele, and bootstrap values are shown. Identical or similar colors indicate identity or similarities of the (GA)z mixed part of D6S2878.

### Figure 2

Pylogenetic analysis of Mamu-DRB exon 2 with the D6S2878 sequences superimposed. The tree was rooted by including the Caja-DRB*W1601 allele, and bootstrapping values are shown. Identical or similar colors indicate identity or similarities of the D6S2878 composition. Mamu-DRB1*0306/03026 and -DRB1*0403/0417, respectively, have nucleotide substitutions outside exon 2.

### Example 1

### Materials and Methods

### Samples

The 167 rhesus monkeys analyzed, housed at BPRC's breeding colony, originated mostly from India, but some are also of Burmese or Chinese origin. Seven of these animals are completely homozygous for their MHC region and derived from consanguineous matings; two additional animals are homozygous for their Mamu-A, -B, and -DR serotypes. Genomic DNA of human individuals or rhesus macaques was extracted from EDTA blood samples or from immortalized B-cell lines using a standard salting out procedure. Of the 160 human samples tested, 64 were HLA-DRB homozygous, 17 of which belong to a thoroughly characterized homozygous typing cell panel of the XIV International Histocompatibility Workshop, 2005.

### STR-DRB genotyping

The relevant DNA segment in rhesus macaques was amplified with a forward primer located at the end of exon 2 (5'Mamu-DRB-STR: TTC ACA GTG CAG CGG CGA GGT) and 2 labeled reverse primers in intron 2 (3'Mamu-DRB-STR_VIC: ACA CCT GTG CCC TCA GAA CT and 3'Mamu-DRB-STR_FAM_1007: ACA TCT GTG TCC TCA GAC CT). For human samples a labeled forward primer located at the end of exon 2 (5'HLA-DRB-STR VIC: GAG AGC TTC ACA GTG CAG CG) and one reverse primer in intron 2 (3'HLA-DRB-STR: GAG AGG ATT CTG AAT GCT CA) was used. The labeled primers were synthesized by Applied Biosystems (Foster City, USA) and the unlabeled primers by Invitrogen (Paisley, Scotland). The PCR reaction for rhesus macaques was performed in a 25 ml reaction volume containing 1 unit of Taq polymerase (Invitrogen, Paisley, Scotland) with 0.6 mM of the unlabeled forward primer, 0.4 mM of the VIC labeled reversed primer, 0.2 mM of the FAM labeled reversed primer, 2.5 mM MgCl2, 0,2 mM of each dNTP, 1 x PCR buffer II (Invitrogen, Paisley, Scotland) and 100 ng DNA.
The PCR mixture for the human STR amplification was the same as that used for rhesus macaques with 0.1 mM of the VIC labeled forward primer and 0.1 mM of the unlabeled reverse primer. The cycling parameters for both amplifications were a 5 min 94°C initial denaturation step, followed by 5 cycles of 1 min at 94°C, 45 s at 58°C, and 45 s at 72°C. Then the program was followed by 25 cycles of 45 s at 94°C, 30 s at 58°C and 45 s at 72°C. A final extension step was performed at 72°C for 30 min. The amplified DNA was prepared for genotyping according to the manufacturer's guidelines and analyzed on an ABI 3130 genetic analyzer (Applied Biosystems). STR analysis was performed with the Genemapper program (Applied Biosystems) and all samples were analyzed at least twice.

### PCR, cloning and sequencing

Seventy-five different Mamu-DRB alleles and 38 HLA-DRB alleles were sequenced from exon 2 to intron 2 including the microsatellite with a generic 5' DRB-exon 2 primer CGT GTC CCC ACA GCA CGT TTC together with the same 3' primers as used for DRB-STR genotyping but without label. The PCR reactions for rhesus monkey and human DRB were performed in a 100 ml volume containing 4 units of Taq polymerase (Invitrogen, Paisley, Scotland) with 0.2 mM of each primer, 2.5 mM MgCl2, 0,2 mM of each dNTP, 1 x PCR buffer II (Invitrogen, Paisley, Scotland) and 200 ng DNA. The cycling parameters were the same as described for STR-DRB genotyping. The resulting amplicons were cloned and sequenced as described recently (Doxiadis et al. 2006. Immunogenetics 58: 259-268; Penedo et al. 2005. Immunogenetics 57, 198-209). The resulting sequences were analyzed using the Sequence Navigator program (Applied Biosystems).

### Phylogenetic analyses

Multiple sequence alignments of exon 2 of human and rhesus macaque -DRB sequences were created using MacVector™ version 8.1.1 (Oxford Molecular Group) and phylogenetic analyses was hen performed using PAUP version 4.0b.10 (Swafford 2002. PAUP*.Phylogenetic Analysis Using Parsimony (*and Other Methods). Version 4 (Sinauer Associates, Sunderland, Massachusetts)). Pairwise distances were calculated using Kimura-2 parameter and the neighbor-joining method for creating a phylogram. Confidence estimates of grouping were calculated according to the bootstrap method generated from 1,000 replicates.

### Results

### Mamu-DRB region configuration definition by microsatellite markers

The rhesus macaques studied are part of a large self-sustaining colony that has been thoroughly pedigreed based on segregation of serologically and molecularly defined markers (Doxiadis et al. 2001. Immunol Rev 183: 76-85; de Groot et al. 2004. J Immunol 172: 6152-7). The current panel covers 22 different region configurations characterized by unique combinations from two to six distinct Mamu-DRB genes (Table 1). Limited allelic polymorphism is detected within region configurations # 1, 11, 15, 18, 19, and 21. To denote allelic variation observed, for instance, in region configuration #1, the relevant haplotypes have been designated 1a and 1b (Table 1). The gene frequencies of the different region configurations as encountered in our population of animals are provided as well, in concert with the number of animals tested. Some haplotypes are frequently observed whereas others appear to be rare (Table 3).
Nearly all Mamu-DRB loci/lineages possess the D6S2878 microsatellite, and the relevant DNA segment could be amplified by means of the unique primer set developed for this protocol (Table 1). Amplification failures have only been observed for a few DRB6/DRBW pseudogenes for which the corresponding amplicons could be scarcely detected, most probably due to primer inconsistencies. The overall results illustrate, however, that the lengths of the amplified STR products are highly variable and range from 153 to 293 bp (Table 1). Additionally, various STR markers seem to be predictive for the presence of a particular Mamu-DRB allele and, moreover, family studies demonstrated that they segregate in a Mendelian manner. Subsequent sequencing of the DNA segment ranging from exon 2 to intron 2 was conducted to unequivocally link each D6S2878 allele to an individual DRB gene/allele. This approach proved that diverse STR lengths could distinguish even highly similar alleles, differing for only one or two nucleotides. An instance is provided by the Mamu-DRB1*07032 and 07033 alleles, which are part of the two haplotypes belonging to configuration #18 (Table 1).
On average, most of the STRs linked to an individual allele appear to be rather conservative in composition and length. In the case of the frequently observed haplotype 11a (Table 3), for example, no length variability for both DRB1 gene-associated D6S2878 alleles is observed, which also holds true for the STR that is linked to DRB5*0301 as seen in haplotype 1a (Table 1). The DRB1*0406-linked STR of the latter configuration, however, may slightly vary in length. Such differences, as can be seen for example in haplotype 12, are reproducible, and do segregate in families with a particular haplotype as defined by serological methods (Bontrop et al. 1995. Immunol Rev 143: 33-62). As each region configuration is composed of an exclusive combination of different Mamu-DRB genes, the most essential conclusion that can be drawn is that the combination of STR markers appears to be unique for a given region configuration/haplotype.

### HLA-DRB haplotype definition by microsatellite analysis

Genotyping for the highly divergent (GT)x(GA)y microsatellite allows speedy and accurate DR haplotyping in rhesus macaques, a species known to possess a high number of region configurations; these display, however, low levels of allelic variation. Next, it was investigated whether the same approach would work in a species, that possesses a relatively low number of region configurations that parade abundant levels of allelic polymorphism. Therefore, in the first instance, a thoroughly characterized panel of 160 unrelated human samples of Caucasoid origin was chosen to conduct such an analysis. This selection also comprised 64 homozygous typing cells, facilitating the simple definition of haplotypes. Furthermore, the panel included the five known HLA-DRB region configurations, designated DR8, DR1, DR51, DR52, and DR53 (Schreuder et al. 2005. Tissue Antigens 65: 1-55), but also covered the 13 most common DR serotypes present in the Caucasoid population, differing for their DRB1 alleles (Table 2). To denote allelic variation as observed within some serotypes, the different DRB haplotypes have been designated a, b, c and so on (Table 2). The number of samples tested per haplotype, as well as the gene frequencies of the serotypes (Marsh et al. 2000. The HLA FactsBook (Academic Press, London, UK, San Diego)), have also been summarized (Table 3).

Again, a specially designed generic primer pair allowed amplification of the relevant intron 2 segment for virtually all HLA-DRB genes/alleles. Amplification failed only for one particular HLA-DRB5 allele present on DR16 haplotypes. This may be due to a mutated primer site. Subsequent extensive sequencing of exon 2-intron 2 DNA segments verified for each of the HLA-DRB alleles the unique linkage to its adjacent D6S2878 allele. The lengths of the respective repeats are highly polymorphic in the human population as well and range from 135 to 220 bp (Table 2). Moreover, STR lengths are highly predictive for the presence of individual HLA-DRB alleles, as was also shown earlier for rhesus monkeys. Again, differential STR lengths can make the distinction between highly similar -DRB alleles, differing for a few nucleotides: for example in the case of DRB1*0801(03) and DRB1*080302 (Table 2).

The DR53 region configuration contains a pseudogene, named HLA-DRB7, which is absent in macaques. This pseudogene displays no allelic variation, and indeed the associated D6S2878 is invariant in length independent of the adjacent -DRB1 allele. Thus, the DRB7-associated STR typifies all the haplotypes belonging to the DR53 region configuration (Table 2). The same holds true for the DRB6 allele and its adjacent STR, which are characteristic for the DR1 region configuration, covering the DR1 and DR10 serotypes. As observed in rhesus macaques, some HLA-DRB alleles appear to be associated with multiple D6S2878 variants. A case is provided by the RLA-DRB1*140101 allele observed within the DR14 haplotypes, but some of the repeats grouping in the DR11a and DR13c family of haplotypes also show length differences (Table 2). Family analyses are needed to demonstrate their segregation. Most of the human repeats, however, appear to be conservative in composition and length, and are linked to an individual allele. Within this panel of 30 different HLA-DRB haplotypes, all but two can be readily dissected based on by their D6S2878 profiles. Thus, complex DR region configuration/haplotype information, as present in at least two populations of primate species, is readily obtained and defined based on D6S2878 genotyping after a simple amplification protocol conducted with one primer set.

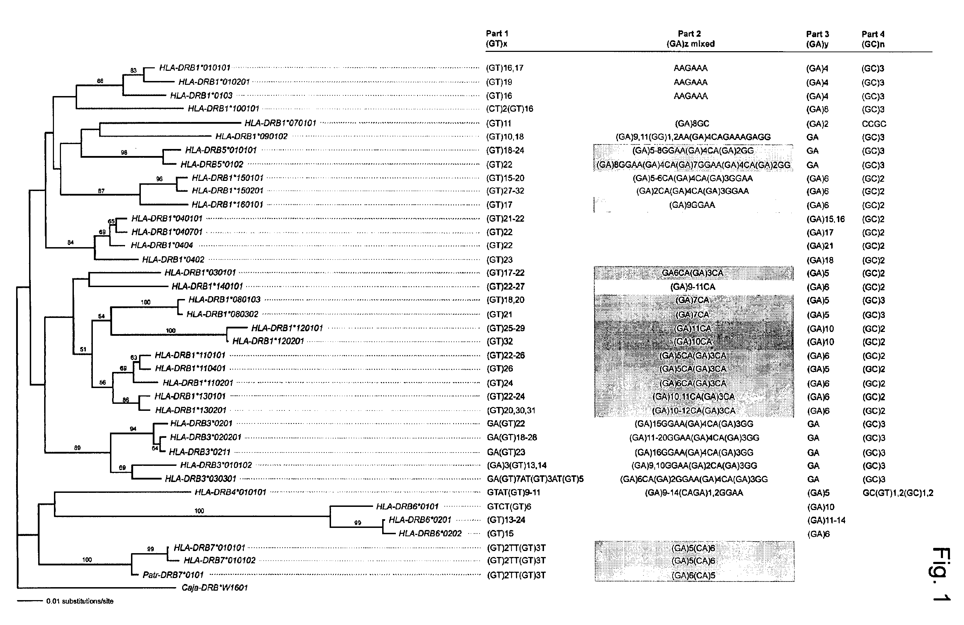

Genetic stability and evolutionary history of the STR-DRB complex The D6S2878 microsatellite has a composite character in primates (Riess et al. 1990. Immunogenetics 32: 110-6; Bergstrom et al. 1999. Am J Hum Genet 64: 1709-18; Kriener et al. 2000. Immunogenetics 51: 169-78; Trtkova et al. 1995. Mol Phylogenet Evol 4: 408-19) and phylogenetic comparisons of different DRB1 sequences obtained from humans and chimpanzees indicated that the ancestral structure most likely must have been a (GT)x(GA)y dinucleotide repeat. The HLA-DRB1 associated microsatellite comprises three sections exhibiting different evolutionary stabilities. The 5'(GT)x repeat represents the longest segment and evolves most rapidly, which is a known feature for long, uninterrupted dinucleotide repeats. The middle section or the (GA)z part is shorter and interrupted; its constellation appears to correlate well with different lineages/loci and its length seems to segregate with specific DRB1 alleles. The length of the 3'(GA)y part appears to be specific for a certain DRB lineage/locus (Bergstrom et al. 1999. Am J Hum Genet 64: 1709-18). All HLA-DRB exon 2 sequences described in this study have been subjected to phylogenetic analyses, on which the D6S2878 sequences have been superimposed (Fig. 1). As can be seen, this report extends the knowledge on this particular microsatellite but also underscores its compound character (Epplen et al. 1997. Hum Genet 99: 399-406; Bergstrom et al. 1999. Am J Hum Genet 64: 1709-18). Additionally, a short dinucleotide (GC)1-3 part could be observed at the 3'end of the microsatellite of all HLA-DRB genes except for those belonging to the DRB6 and DRB7 pseudogenes. The variation seen in this newly recognized section of the microsatellite seems to be prognostic for certain HLA-DRB lineages or loci, respectively. The 5'(GT)x part is indeed the most polymorphic, which may especially in the case of fairly long (GT)x repeats evolve faster than the mutation rate operative on exon 2 itself (Fig. 1). This phenomenon, already described for DQCAR (Lin et al. 1998. Tissue Antigens 52: 9-18) could explain why, for instance, the HLA-DRB1*1302 allele is associated with a STR displaying length variation (Table 2).

The ancient HLA-DRB6 and -7 pseudogenes appear to miss the middle, interrupted (GA)z or the (GA)y part, respectively. The HLA-DRB6*0101 gene harbors a genetically stable D6S2878 showing no length differences at all. This is most probably due to its composition represented by a short and interrupted 5'(GT) and 3'(GA) part (Petes et al. 1997. Genetics 146: 491-8; Wierdl et al. 1997.Genetics 146: 769-79). For HLA-DRB6*0201 the opposite is true, and the long and uninterrupted (GT)x and (GA)y parts are indicative for unstable STR lengths (Table 2 and Fig. 1) (Jin et al. 1996. Proc Natl Acad Sci U S A 93: 15285-8).

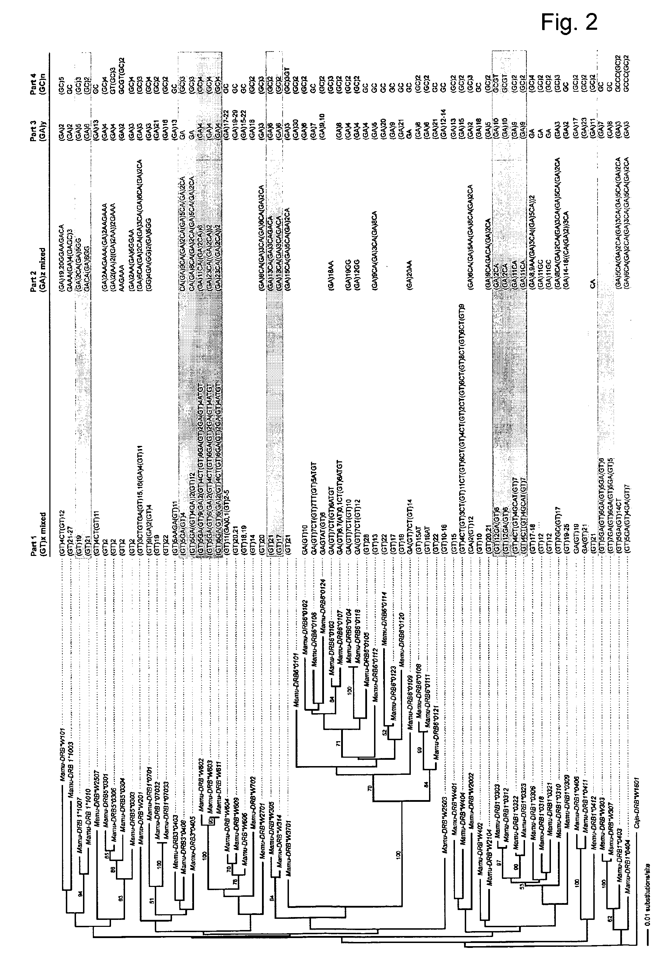

The Mamu-DRB exon 2 sequences have also been subjected to phylogenetic analyses and the genetic composition of the D6S2878 sequences has been superimposed (Fig. 2). Like in humans, D6S2878 has a compound character. The newly described fourth 3'(GC) dinucleotide part is also present in rhesus macaques with repeat lengths ranging from 1 to 5. Comparable to the human situation, Mamu-DRB6 alleles form a distinct clade in the phylogenetic tree. Since the DRB6 locus is thought to predate the divergence of Old World monkeys, great apes, and homonoids, it is not surprising that the same ancestral (GT)x(GA)y structure was detected in rhesus monkeys just as in humans. In contrast to the human microsatellite, some Mamu-DRB lineages/loci are characterized by a multipart (GT)x and/or a (GA)z middle segment. In general, the rhesus macaque STR appears to be even more complex than its human equivalent. This multifaceted composition seems to correlate with the high number of DRB region configuration/haplotype diversity observed in rhesus macaques. Despite the complex microsatellite patterns observed in the rhesus macaque, the composition and length of repeats associated with known Mamu-DRB alleles, are as expected, highly similar. The rhesus macaque DRB region has been subject to several rounds of duplication and contraction processes. For that reason, it is difficult to understand which highly related genes located on different region configurations/haplotypes represent separate loci or whether these sequences have an allelic affiliation. This microsatelite will be helpful in sorting out such genetic relationships.

### Discussion

Microsatellite D6S2878 is considered to represent a promising marker for the development of a quick and accurate DRB haplotyping protocol in primate species, on the condition that one single set of informative primers can be developed. Indeed, a specifically designed primer pair allowed amplification of the relevant intron 2 segment for virtually all HLA- as well as Mamu-DRB genes/alleles. For both species, amplification artifacts have rarely been observed and such types of problems are easily to overcome by designation of specific primers that can be added to the same reaction mixture. The D6S2878 STR was proven to be highly variable in length, not only in humans but also in the rhesus macaque, thus verifying this microsatellite as a useful marker for DRB typing of both species. Phylogenetic analyses of human as well as rhesus macaque exon 2 sequences have been performed and compared with microsatellite composition. For humans, the evolutionary relationships of exon 2 and the adjacent microsatellite seem to segregate closely together (Fig. 1). In rhesus macaques, the microsatellite composition was far more variable than in humans and a comparison of microsatellite and exon 2 phylogeny does not always seem to match the microsatellie composition (Fig. 2). One explanation for these results may be that rhesus macaque DRB loci/lineages are much older than their human equivalents, and this can be the reason for the higher diversity of the adjacent microsatellite as well. Furthermore, preliminary results of intron sequences illustrate that some of the Mamu-DRB sequences that are considered alleles of a given locus probably represent monomorphic loci themselves. However, truly allelic variants manifest the reliability of the comparison of exon 2 and the D6S2878 marker not only in humans but also in rhesus macaques.

Microsatellite typing was, in the first instance performed on large human and rhesus macaque panels for which the typing information was known. The most essential conclusion to be drawn (Table 1 and 2) is that in both species the combination of STR markers appears to be unique for a given haplotype. Within the rhesus macaque panel of 31 haplotypes all could be defined unambiguously, within the human panel of 30 haplotypes all but two could be thus defined. As stated earlier, ambiguities can easily be solved by development of additional primer pairs. As a control, a blind test was performed with 47 human and 26 rhesus monkey samples. More than 90% of the samples were scored correctly for their Mhc-DRB haplotypes. Some of the samples were not scored properly because they contained allotypes that were not present in the original test panel. Thus, this D6S2878 typing protocol provides a highly reliable method for Mamu- and HLA-DRB haplotyping, with its main advantage being simplicity and speed. Since differently labeled primers can be used for microsatellite typing, multiplexing is possible and 96 samples or even more can be analyzed within one test panel. The simplicity of the test is especially useful for Mamu-DRB haplotyping, which is otherwise extremely time consuming due to the unprecedented high number of different -DRB region configurations. For the human situation, this approach is very helpful in the analysis of large amounts of samples, as they are needed, for example, in population and/or disease association studies. Furthermore, this method may also be of use in forensic medicine as well as in paternity-testing protocols. Additionally, high-resolution -DRB haplotyping will simplify donor-recipient matching in organ as well as bone marrow transplantation. As the D6S2878 STR is an old entity, it may also be used to study other populations of primate species.

The evolutionary stability of this microsatellite has been a matter of debate (Epplen et al. 1997. Hum Genet 99: 399-406; Riess et al. 1990. Immunogenetics 32: 110-6; Bergstrom et al. 1999. Am J Hum Genet 64: 1709-18; Trtkova et al. 1995. Mol Phylogenet Evol 4: 408-19; Maueler et al. 1999. Gene 226: 9-23). The HLA-DRB7 associated D6S2878 allele is especially remarkable, as this repeat has the shortest (GT)x as well as the (GA)y part and is highly stable in length, showing no polymerase slippage at all. This is in accordance with the fact that short and/or interrupted repeats are more stable than long, uninterrupted dinucleotides (Petes et al. 1997. Genetics 146: 491-8; Wierdl et al. 1997. Genetics 146: 769-79; Jin et al. 1996. Proc Natl Acad Sci U S A 93: 15285-8). To what extent the repeat composition may have a direct or indirect influence on the low mutation rate of the adjacent exon 2 segment of the DRB7 pseudogene is not well understood at present. It has been proposed, for example, that microsatellites near genes may increase and probably also decrease local mutation rates (Vowles and Amos 2004. PLoS Biol 2: E199). Interestingly, exon 2 of the DRB7 pseudogene, present on the only shared DR region configuration of humans and chimpanzees, is highly conserved between both species. Moreover, the D6S2878 sequence is completely identical (Fig. 1). It has been suggested that the intron 2 segment containing the (GT)x(GA)y repeat may bind a zinc-dependent protein and forms non B-DNA structures; thus, functionality of these so-called 'junk' DNA sequences cannot be ruled out and should be subjected to further analysis (Maueler et al. 1999. Gene 226: 9-23).

**Table 1 Mamu-DRB haplotypes defined by exon 2 sequencing and D6S2878 genotyping**

| hapl | 1^{st} *DRB* locus 6^{th} DRB | STR locus STR | 2^{nd} *DRB* locus | STR | 3^{rd} DRB locus | STR | 4^{th} *DRB* locus | STR | 5^{th} *DRB* locus | STR |
|---|---|---|---|---|---|---|---|---|---|---|
| 1a | *DRB1*0406* | 205, 207, 211 | *DRB5*0301* | 169 | | | | | | |
| 1b | *DRB1*0411* | 223 | *DRB5*0304* | 153 | | | | | | |
| | | | | | | | | | | |
| 2 | *DRB3*0403* | 192 | *DRB*W305* | 227 | | | | | | |
| | | | | | | | | | | |
| 3^{a} | *DRB1*0412* | 199 | *DRB*W3701* | 235 | | | | | | |
| | | | | | | | | | | |
| 4^{a} | *DRB1*1010* | 205 | *DRB6*0121* | 216 | | | | | | |
| | | | | | | | | | | |
| 5^{a} | *DRB3*0408* | 203 | *DRB*W404* | 293 | | | | | | |
| | | | | | | | | | | |
| 6^{a} | *DRB3*0411* | 190 | *DRB*W314* | 219 | | | | | | |
| | | | | | | | | | | |
| 7^{b} | *DRB4*0102* | 291 | *DRB5*0306* | 173 | | | | | | |
| | | | | | | | | | | |
| 8^{b} | *DRB1*0321* | 181 | *DRB1**0322/3 | 209 | | | | | | |
| | | | | | | | | | | |
| 9^{b} | *DRB1*0321* | 181 | *DRB1*0322*/*3* | 209 | *DRB1*1003^{c}* | 175? | | | | |
| | | | | | | | | | | |
| 10^{b} | *DRB1*0309* | 229 | *DRB*W2507* | 181 | *DRB6*0109* | ? | | | | |
| | | | | | | | | | | |
| 11a | *DRB1*0303* | 195 | *DRB1*1007* | 197 | *DRB6*0103* | (202-206) | | | | |
| 11b | *DRB1*0312* | 195 | *DRB1*1007* | 201 | *DRB6*0103* | ? | | | | |
| 11c | *DRB1*0306* | 226 | *DRB1*1007* | 197 | *DRB6*0103* | ? | | | | |
| 11d | *DRB1*0306* | 226-230 | *DRB1*1003* | 199-213 | *DRB6*0107* | 182 | | | | |
| | | | | | | | | | | |
| 12 | *DRB1*0309* | 233-247 | *DRB6*0101* | ? | *DRB*W201* | 263 (261, 267) | | | | |
| | | | | | | | | | | |
| 13 | *DRB6*0112* | 214 | *DRB*W2501* | 218? | *DRB*W2002* | 218 | *DRB6*0106?* | 205? | | |
| | | | | | | | | | | |
| 14 | *DRB6*0114* | 218 | *DRB*W303* | 193 | *DRB*W401* | 187 | | | | |
| | | | | | | | | | | |
| 15a | *DRB*/**0403* | 229 | *DRB6*0107* | 184 | *DRB*W501* | ? | *DR86*?* | 208 | | |
| 15b^{b} | *DRB1*0403* | 229 | *DRB6*0107* | 183 | *DRB*W502* | ? | *DRB6*0120* | 208 | | |
| | | | | | | | | | | |
| 16 | *DRB1*0404* | 225 | *DRB6*0102* | 164 | *DRB*W307* | 195 | *DRB*W702* | 188 | | |
| | | | | | | | | | | |
| 17 | *DRB1*0701* | 197 | *DRB3*0405* | 237 | *DRB5*0303* | 169 | *DRB6*0123* | 184 | | |
| | | | | | | | | | | |
| 18a^{a} | *DRB1*0326* | 214 | *DRB1*07032* | 211 | *DRB6*0124* | 170 | *DRB*W2603* | 177 | *DRB6*0120* | ? |
| 18b^{b} | *DRB1*0326* | 214 | *DRB1*07033* | 207 | *DRB6*0124* | 168 | *DRB*W2604* | 177 | *DRB6*0120* | 208 |
| | | | | | | | | | | |
| 19a | *DRB1*0318* | 181 | *DRB6*0105* | 190, 192 | *DRB*W604* | 201, 205 | *DRB*W603* | 275, 277 | *DRB6*0104*(+ALU) | 222 |
| (220) | | | | | | | | | | |
| 19b | *DRB1*0318* | 181 | *DRB6*0105* | 194 | *DRB*W604* | 197 | *DRB*W611* | 275 | *DRB6*01?* | 222 |
| | | | | | | | | | | |
| 20^{a} | *DRB3*0403* | 182 | *DRB*W402* | 185 | *DRB*W2701* | 227 | *DRB6*0116* | 206 | *DRB6*0107* | 182 |
| | | | | | | | | | | |
| 21a | *DRB6*0111* | 178 | *DRB*W606* | 211,213, 197 | *DRB*W2104* | 211, 213 | *DRB*W2603* | 177, 187 | *DRB6*0122* | 236 |
| 21b^{b} | *DRB6*0108* | 176 | *DRB*W606* | 213 | *DRB*W2104* | 213 | *DRB*W2604* | 177 | *DR86*0122* | 232 |
| | | | | | | | | | | |
| 22 | *DRB1*0310* | 241 | *DRB6*0118* | 210 | *DRB*W101* | 219,223 | *DRB*W602* | 279 (285) *DRB*W609* | 219 (223) | |
| | *DRB6*0106* | 192 | | | | | | | | |

| | | | | | | | | | | |
|---|---|---|---|---|---|---|---|---|---|---|
| ^{a}Animals with Chinese, ^{b}animals with Burmese origin. *^{c}DRB1*1003* belong to haplotype 8 or 9; 0 STR length observed in some (1-3) animals; ? STR not or rarely detected but presence of gene ascertained by sequencing; 205?, 175? STR's detected but not confirmed by sequencing | | | | | | | | | | |

**Table 2 HZA-DRB haplotypes defined by sequencing and D6S2878 genotyping**

| Haplotype | *DRB1* locus | | STR | 2^{nd} DRB locus | | | STR | | 3^{rd} *DRB* locus | STR | |
|---|---|---|---|---|---|---|---|---|---|---|---|
| DR8 | DR8a | *DRB1*0801(03)* | | 170 (172) | | | | | | | |
| | DR8b | *DRB1*080302* | | 176 | | | | | | | |
| | | | | | | | | | | | |
| DR1 | DR1a/DR103 | *DRB1*010101*/*0103* | 154 (156) | | *DRB6*010101* | 136 | | | | | |
| | DR1b | *DRB1*010201* | | 160 | | *DRB6*0101* | | 136 | | | |
| | | | | | | | | | | | |
| | DR10 | *DRB1*100101* | | 161 | | *DRB6*0101* | | 136 | | | |
| | | | | | | | | | | | |
| DR52 | DR17a | *DRB1*030101* | | 172 | | *DR83*010101(2)* | | 180 | | | |
| | DR17b | *DRB1*0301* | | 178-184 | | *DRB3*0202(01* | | 208 (206,210) | | | |
| | | | | | | | | | | | |
| | DR11a | *DRB1*110101* | | 182-192 | | *DRB3*020201* | | 206-218 | | | |
| | DR11b | *DRB1*110201* | | 186 | | *DRB3*020201* | | 214 | | | |
| | DR11c | *DRB1*110401* | | 188 | | *DRB3*020201* | | 208(210) | | | |
| | | | | | | | | | | | |
| | DR12a | *DRB1*120101* | | 200 | | *DRB3*010102* | | 180 | | | |
| | DR12b | *DRB1*1201* | | 206 (208) | | *DRB3*02(0201)* | 210,212 | | | | |
| | DR12c | *DRB1*120201* | | 216 | | *DRB3*030101* | | 186 | | | |
| | | | | | | | | | | | |
| | DR13a | *DRB1*1301(01)* | | 194 | | *DR83*0101(02)* | | 180 | | | |
| | DR13b | *DRB1*1301(01)* | | 194, 196 | | *DRB3*02(0201)* | | 204-214 | | | |
| | DR13c | *DRB1*1302(01)* | | 188,206-218 | | *DRB3*0301*/*0101* | | 186 | | | |
| | | | | | | | | | | | |
| | DR14a | *DRB1*140101* | | 192 | | *DRB3*0211* | | 212 | | | |
| | DR14b | *DRB1*140701* | | 186, 188, 196 | | *DRB3*020201* | | 210 | | | |
| | DR14c | *DRB1*140101* | | 198 | | *DRB3*0201* | | 214 | | | |
| | | | | | | | | | | | |
| DR53 | DR4a | *DRB1*0401(01)* | | 180,182, 184 | | *DRB4*(010101)* | | 178, 180, (176, 182/4) | *DRB7*010101* | | 135 |
| | DR4b | *DRB1*0402* | | 194 | | *DRB4*010101* | | 176 | *DRB7^{*}010101* | | 135 |
| | DR4c | *DRB1*0404* | | 192 (196) | | *DRB4*010101* | 178 | | *DRB7*010101* | 135 | |
| | DR4d | *DRB1*040701* | | 184 | | *DRB4*010101* | | 178 | *DRB7*010101* | | 135 |
| | DR4e | *DRB1*0408* | | 180 | | *DRB4*010101* | | 178 | *DRB7*010101* | | 135 |
| | | | | | | | | | | | |
| | DR7 | *DRB1*070101* | | 149 | | *DRB4*010101* | | 170, 176, 180 | *DRB7*010101*/*2* | | 135 |
| | | | | | | | | | | | |
| | DR9 | *DRB1*09(0102)* | | 170 (196) | | *DRB4*010101* | 180 | | *DRB7*010101* | 135 | |
| | | | | | | | | | | | |
| DR51 | DR15a | *DRB1*150101* | | 186 | | *DRB5*0101(01)* | | 180 | *DRB6*(0201)* | | 154 |
| | DR15b | *DRB1*150101* | | 186, 190 (184, 188) | | *DRR5*0101(01)* | 184 (190) | *DRB6*(0201)* | 152 | | |
| | DR15c | *DRB1*150201* | | 202-208 | | *DRB5*0102* | | 220 | *DRB6*(0201)* | | 174 |
| | | | | | | | | | | | |
| | DR16 | *DRB1*160101* | | 178 | | *DRB5*?* | | 184 | *DRB6*0202* | | 144 |

| | | | | | | | | | | | |
|---|---|---|---|---|---|---|---|---|---|---|---|
| () indicates STR lengths detected only once or twice | | | | | | | | | | | |

**Table 3. Gene frequencies and numbers (#) of Mamu- and HLA-DRB haplotypes tested by D6S2878**

| ***Mamu*** | | | ***HLA*** | | |
|---|---|---|---|---|---|
| **hapl^{a}** | **#** | **gf^{b} (2n = 240)** | **hapl^{a}** | **#** | **gf^{c}** |
| 1a | 43 (4)^{d} | 0,104 | DR8a | 5 (2)^{d} | 0.039 |
| 1b | 4 | 0,004 | DR8b | 2 (2) | |
| 2 | 20 (2) | 0,017 | DR1a-DR103 | 42 (20) | 0.094 |
| 3 | 1 | 0,004 | DR1b | 8 (2) | |
| 4 | 2 | 0,004 | DR10 | 4 | 0.013 |
| 5 | 2 | 0,004 | DR52-DR17a | 34 (16) | 0.111 |
| 6 | 2 | 0,004 | -DR17b | 12 (6) | |
| 7 | 1 | 0,004 | -DR11a | 25 (2) | |
| 8 | 3 | 0,004 | -DR11b | 2 (2) | 0.134 |
| 9 | 1 | 0,004 | -DR11c | 6 (6) | |
| 10 | 4 | 0,008 | -DR12a | 1 | |
| 11a | 32 | 0,063 | -DR12b | 5 (2) | 0.023 |
| 11b | 7 | 0,017 | -DR12c | 1 | |
| 11a or | | | -DR13a | 13 (6) | |
| b^{e} | | 0,083 | | | 0.102 |
| 11c | 7 (4) | 0,004 | -DR13b | 16 (6) | |
| 11d | 21 | 0,096 | -DR13c | 12 (4) | |
| 12 | 58 (2) | 0,163 | -DR14a | 1 | |
| 13 | 10 | 0,008 | -DR14b | 4 (2) | 0.032 |
| 14 | 7 (4) | 0,021 | -DR14c | 2 (2) | |
| 15a | 7 (2) | 0,033 | DR53-DR4a | 23 (C) | |
| 15b | 2 | 0,008 | -DR4b | 2 (2) | |
| 16 | 3 | 0,029 | -DR4c | 11 (6) | 0.128 |
| 17 | 7 | 0,025 | -DR4d | 2 | |
| 18a | 3 | 0,004 | -DR4e | 1 | |
| 18b | 2 | 0,004 | -DR7 | 28 (12) | 0.132 |
| 19a | 5 | 0,071 | -DR9 | 10 (2) | 0.014 |
| 19b | 3 | 0,013 | DR51-DR15a1 | 5 | |
| 20 | 1 | 0,008 | -DR15a2 | 39 (16) | 0.107 |
| 21a | 20 (2) | 0,050 | -DR15b | 4 (2) | |
| 21b | 2 | 0,004 | -DR16 | 2 (2) | 0.036 |
| 22 | 14 (2) | 0,042 | | | |
| other | n.t. | 0,092 | others | n.t. | 0.035 |
| total | 294 | 1,000 | total | 322 | 1.000 |

| | | | | | |
|---|---|---|---|---|---|
| *^{a}Mhc-DRB* haplotypes correspond to those defined in Table 1 and 2, respectively. ^{b}Gene frequencies (gf) of *Mamu-DRB* haplotypes of the rhesus macaque breeding colony. ^{c}Gene frequencies (gf) of caucasoid *HLA-DRB* haplotypes according to Marsh and coworkers (39). ^{d} () refer to # of homozygous typing cells included in the total #. ^{e}Haplotypes 9a and b could only be descriminated by D6S2878 genotyping and sequencing; n.t. not tested. | | | | | |

## Claims

1. Method for determining a DRB haplotype in a sample comprising nucleic acid from a mammalian cell, said method comprising
amplifying at least an intron 2 microsatellite-containing part of at least one DRB-gene from said nucleic acid;
determining the type of intron 2 microsatellite present in said at least one DRB-gene; and
determining the DRB haplotype from said type of intron 2 microsatellite present in said at least one DRB-gene.

2. Method according to claim 1, wherein said type of intron 2 microsatellite is determined based on the length of said microsatellite.

3. Method according to claim 1 or claim 2, wherein the DRB-haplotype is determined by comparing the detected types of intron 2 microsatellite with a reference.

4. Method according to claim 3, wherein said reference comprises a database of DRB haplotypes correlated with the associated intron 2 microsatellite types.

5. Method according to claim 4, wherein said database is present in an electronic storage device.

6. Method according to any of the previous claims, wherein said amplification comprises providing said sample with a primer pair that spans the region containing said intron 2 microsatellite.

7. Method according to claim 6, wherein said primer pair comprises a first primer of which the nucleotide sequences correspond to a conserved nucleotide sequence that is present in a position that is adjacent to the microsatellite in intron 2, and a second primer of which the nucleotide sequences corresponds to a conserved sequence that is present on the opposite side of said microsatellite sequence, relative to the first primer.

8. Method according to claim 6 or 7, wherein one or more of the primers used is labelled.

9. Method according to any of the previous claims, wherein the nucleic acid from a mammalian cell comprises genomic DNA.

10. Method according to any of the previous claims, wherein the sample is derived from a primate.

11. A kit for determining a DRB haplotype in a cell, comprising means for amplifying at least an intron 2 microsatellite-containing part of at least one DRB-gene

12. Kit according to claim 11, comprising a pair of primers.

13. Kit according to claim 12, wherein a first primer of said primer pair comprises sequences selected from a conserved region spanning the 3' end of exon 2 and the 5'start of intron 2 of a DRB gene, while a second primer comprises sequences selected from a conserved region that is present on the opposite side of said microsatellite sequence, relative to the first primer.

14. Kit according to any one of claims 11-13, wherein a 5' primer comprises the nucleotide sequence of SEQ ID NO 1; and a 3' primer comprises the nucleotide sequence of SEQ ID NO 3.

15. Kit according to any one of claims 11-13, wherein a 5' primer comprises the nucleotide sequence of SEQ ID NO 2; and a 3' primer comprises the nucleotide sequence of SEQ ID NO 4 or SEQ ID NO 5.

16. Kit according to any one of claims 11-13, wherein a 5' primer comprises the nucleotide sequence of SEQ ID NO 6; and a 3' primer comprises the nucleotide sequence of SEQ ID NO 3, SEQ ID NO 4 or SEQ ID NO 5.

17. Use of a method for determining a DRB haplotype in a sample, said method comprising amplifying at least a microsattellite-comprising region of intron 2 of at least one DRB gene, for a genetic application.

18. Use according to claim 17, wherein the genetic application comprises paternity testing.

19. Use according to claim 17, wherein the genetic application comprises a forensic testing.

20. Use according to claim 17, wherein the genetic application comprises tissue typing for transplantation testing.

21. Use according to claim 17, wherein the genetic application comprises testing for chronic or infectious diseases.

22. Us of the kit of any of claims 11-15 in a genetic application comprising paternity testing, forensic testing, tissue typing for transplantation testing, or testing for chronic or infectious diseases.


SEQUENCE LISTING

<110> Stichting Biomedical Primate Research Centre

<120> Means and methods for haplotyping MHC-DRB loci in mammals and
uses thereof

<130> P79234EP00

<140> EP 07105879.6
<141> 2007-04-10

<160> 133

<170> PatentIn version 3.3

<210> 1
<211> 20
<212> DNA
<213> Artificial

<220>
<223> primer 5'HLA-DRB-STR_VIC

<400> 1
gagagcttca cagtgcagcg 20

<210> 2
<211> 21
<212> DNA
<213> Artificial

<220>
<223> 5'Mamu-DRB-STR primer

<400> 2
ttcacagtgc agcggcgagg t 21

<210> 3
<211> 20
<212> DNA
<213> Artificial

<220>
<223> 3'HLA-DRB-STR primer

<400> 3
gagaggattc tgaatgctca 20

<210> 4
<211> 20
<212> DNA
<213> Artificial

<220>
<223> 3'Mamu-DRB-STR_VIC primer

<400> 4
acacctgtgc cctcagaact 20

<210> 5
<211> 20
<212> DNA
<213> Artificial

<220>
<223>  3'Mamu-DRB-STR_FAM_1007 primer

<400>  5
acatctgtgt cctcagacct                                                    20


<210>  6
<211>  21
<212>  DNA
<213>  Artificial

<220>
<223>  5'DRB-exon2 primer

<400>  6
cgtgtcccca cagcacgttt c                                                  21


<210>  7
<211>  52
<212>  DNA
<213>  Homo sapiens

<400>  7
gtgtgtgtgt gtgtgtgtgt gtgtgtgtgt gtaagaaaga gagagagcgc gc                52


<210>  8
<211>  54
<212>  DNA
<213>  Homo sapiens

<400>  8
gtgtgtgtgt gtgtgtgtgt gtgtgtgtgt gtgtaagaaa gagagagagc gcgc              54


<210>  9
<211>  58
<212>  DNA
<213>  Homo sapiens

<400>  9
gtgtgtgtgt gtgtgtgtgt gtgtgtgtgt gtgtgtgtaa gaaagagaga gagcgcgc          58


<210>  10
<211>  58
<212>  DNA
<213>  Homo sapiens

<400>  10
ctctgtgtgt gtgtgtgtgt gtgtgtgtgt gtgtgtgaga gagagagaga gagcgcgc          58


<210>  11
<211>  48
<212>  DNA
<213>  Homo sapiens

<400>  11
gtgtgtgtgt gtgtgtgtgt gtgagagaga gagagagagc gagaccgc                     48


<210>  12
<211>  68
<212>  DNA

<213>  Homo sapiens

<400>  12
gtgtgtgtgt gtgtgtgtgt gagagagaga gagagagagg aagagagaga cagaaagagg      60

gagcgcgc                                                                68


<210>  13
<211>  90
<212>  DNA
<213>  Homo sapiens

<400>  13
gtgtgtgtgt gtgtgtgtgt gtgtgtgtgt gtgtgtgaga gagagagaga gagagagagg      60

ggaagagaga gacagaaaga gggagcgcgc                                        90


<210>  14
<211>  74
<212>  DNA
<213>  Homo sapiens

<400>  14
gtgtgtgtgt gtgtgtgtgt gtgtgtgtgt gtgtgtgaga gagagaggaa gagagagaca      60

gagagggagc gcgc                                                         74


<210>  15
<211>  92
<212>  DNA
<213>  Homo sapiens

<400>  15
gtgtgtgtgt gtgtgtgtgt gtgtgtgtgt gtgtgtgtgt gtgtgtgtga gagagagaga      60

gagaggaaga gagagacaga gagggagcgc gc                                     92


<210>  16
<211>  116
<212>  DNA
<213>  Homo sapiens

<400>  16
gtgtgtgtgt gtgtgtgtgt gtgtgtgtgt gtgtgtgtgt gtgtgagaga gagagagaga      60

ggaagagaga gacagagaga gagagagagg aagagagaga cagagaggga gcgcgc          116


<210>  17
<211>  78
<212>  DNA
<213>  Homo sapiens

<400>  17
gtgtgtgtgt gtgtgtgtgt gtgtgtgtgt gagagagaga cagagagaga cagagagagg      60

aagagagaga gagagcgc                                                     78


<210>  18
<211>  90
<212>  DNA
<213>  Homo sapiens

<400>  18
gtgtgtgtgt gtgtgtgtgt gtgtgtgtgt gtgtgtgtgt gagagagaga gacagagaga      60

gacagagaga ggaagagaga gagagagcgc                                        90


<210>  19
<211>  96
<212>  DNA
<213>  Homo sapiens

<400>  19
gtgtgtgtgt gtgtgtgtgt gtgtgtgtgt gtgtgtgtgt gtgtgtgtgt gtgtgagaca      60

gagagagaca gagagaggaa gagagagaga gagcgc                                 96


<210>  20
<211>  106
<212>  DNA
<213>  Homo sapiens

<400>  20
gtgtgtgtgt gtgtgtgtgt gtgtgtgtgt gtgtgtgtgt gtgtgtgtgt gtgtgtgtgt      60

gtgtgagaca gagagagaca gagagaggaa gagagagaga gagcgc                     106


<210>  21
<211>  72
<212>  DNA
<213>  Homo sapiens

<400>  21
gtgtgtgtgt gtgtgtgtgt gtgtgtgtgt gtgtgagaga gagagagaga gaggaagaga      60

gagagagagc gc                                                           72


<210>  22
<211>  76
<212>  DNA
<213>  Homo sapiens

<400>  22
gtgtgtgtgt gtgtgtgtgt gtgtgtgtgt gtgtgtgtgt gtgagagaga gagagagaga      60

gagagagaga gagcgc                                                       76


<210>  23
<211>  80
<212>  DNA
<213>  Homo sapiens

<400>  23
gtgtgtgtgt gtgtgtgtgt gtgtgtgtgt gtgtgtgtgt gtgtgagaga gagagagaga      60

gagagagaga gagagagcgc                                                   80


<210>  24
<211>  82
<212>  DNA
<213>  Homo sapiens

<400>  24
gtgtgtgtgt gtgtgtgtgt gtgtgtgtgt gtgtgtgtgt gtgtgagaga gagagagaga      60

gagagagaga gagagagagc gc                                              82

<210>  25
<211>  90
<212>  DNA
<213>  Homo sapiens

<400>  25
gtgtgtgtgt gtgtgtgtgt gtgtgtgtgt gtgtgtgtgt gtgtgagaga gagagagaga      60

gagagagaga gagagagaga gagagagcgc                                        90

<210>  26
<211>  86
<212>  DNA
<213>  Homo sapiens

<400>  26
gtgtgtgtgt gtgtgtgtgt gtgtgtgtgt gtgtgtgtgt gtgtgtgaga gagagagaga      60

gagagagaga gagagagaga gagcgc                                            86

<210>  27
<211>  70
<212>  DNA
<213>  Homo sapiens

<400>  27
gtgtgtgtgt gtgtgtgtgt gtgtgtgtgt gtgtgagaga gagagacaga gagacagaga      60

gagagagcgc                                                              70

<210>  28
<211>  80
<212>  DNA
<213>  Homo sapiens

<400>  28
gtgtgtgtgt gtgtgtgtgt gtgtgtgtgt gtgtgtgtgt gtgtgagaga gagagacaga      60

gagacagaga gagagagcgc                                                   80

<210>  29
<211>  80
<212>  DNA
<213>  Homo sapiens

<400>  29
gtgtgtgtgt gtgtgtgtgt gtgtgtgtgt gtgtgtgtgt gtgtgagaga gagagagaga      60

gacagagaga gagagagcgc                                                   80

<210>  30
<211>  94
<212>  DNA
<213>  Homo sapiens

<400>  30
gtgtgtgtgt gtgtgtgtgt gtgtgtgtgt gtgtgtgtgt gtgtgtgtgt gtgtgagaga      60

gagagagaga gagagacaga gagagagaga gcgc                                   94

<210> 31
<211> 68
<212> DNA
<213> Homo sapiens

<400> 31
gtgtgtgtgt gtgtgtgtgt gtgtgtgtgt gtgtgtgaga gagagagaga cagagagaga 60

gagcgcgc 68


<210> 32
<211> 72
<212> DNA
<213> Homo sapiens

<400> 32
gtgtgtgtgt gtgtgtgtgt gtgtgtgtgt gtgtgtgtgt gagagagaga gagacagaga 60

gagagagcgc gc 72


<210> 33
<211> 74
<212> DNA
<213> Homo sapiens

<400> 33
gtgtgtgtgt gtgtgtgtgt gtgtgtgtgt gtgtgtgtgt gtgagagaga gagagacaga 60

gagagagagc gcgc 74


<210> 34
<211> 98
<212> DNA
<213> Homo sapiens

<400> 34
gtgtgtgtgt gtgtgtgtgt gtgtgtgtgt gtgtgtgtgt gtgtgtgtgt gagagagaga 60

gagagagaga gacagagaga gagagagaga gagagcgc 98


<210> 35
<211> 106
<212> DNA
<213> Homo sapiens

<400> 35
gtgtgtgtgt gtgtgtgtgt gtgtgtgtgt gtgtgtgtgt gtgtgtgtgt gtgtgtgtga 60

gagagagaga gagagagaga cagagagaga gagagagaga gagcgc 106


<210> 36
<211> 110
<212> DNA
<213> Homo sapiens

<400> 36
gtgtgtgtgt gtgtgtgtgt gtgtgtgtgt gtgtgtgtgt gtgtgtgtgt gtgtgtgtgt 60

gtgtgagaga gagagagaga gagacagaga gagagagaga gagagagcgc 110

<210> 37
<211> 80
<212> DNA
<213> Homo sapiens

<400> 37
gtgtgtgtgt gtgtgtgtgt gtgtgtgtgt gtgtgtgtgt gtgtgagaga gagacagaga 60

gacagagaga gagagagcgc 80


<210> 38
<211> 88
<212> DNA
<213> Homo sapiens

<400> 38
gtgtgtgtgt gtgtgtgtgt gtgtgtgtgt gtgtgtgtgt gtgtgtgtgt gtgagagaga 60

gacagagaga cagagagaga gagagcgc 88


<210> 39
<211> 86
<212> DNA
<213> Homo sapiens

<400> 39
gtgtgtgtgt gtgtgtgtgt gtgtgtgtgt gtgtgtgtgt gtgtgtgtgt gtgagagaga 60

gacagagaga cagagagaga gagcgc 86


<210> 40
<211> 86
<212> DNA
<213> Homo sapiens

<400> 40
gtgtgtgtgt gtgtgtgtgt gtgtgtgtgt gtgtgtgtgt gtgtgtgtga gagagagaga 60

cagagagaca gagagagaga gagcgc 86


<210> 41
<211> 90
<212> DNA
<213> Homo sapiens

<400> 41
gtgtgtgtgt gtgtgtgtgt gtgtgtgtgt gtgtgtgtgt gtgtgagaga gagagagaga 60

gagacagaga gacagagaga gagagagcgc 90


<210> 42
<211> 96
<212> DNA
<213> Homo sapiens

<400> 42
gtgtgtgtgt gtgtgtgtgt gtgtgtgtgt gtgtgtgtgt gtgtgtgtga gagagagaga 60

gagagagaga cagagagaca gagagagaga gagcgc 96


<210> 43
<211> 86

<212>  DNA
<213>  Homo sapiens

<400>  43
gtgtgtgtgt gtgtgtgtgt gtgtgtgtgt gtgtgtgtgt gagagagaga gagagagaga      60

cagagagaca gagagagaga gagcgc                                            86


<210>  44
<211>  112
<212>  DNA
<213>  Homo sapiens

<400>  44
gtgtgtgtgt gtgtgtgtgt gtgtgtgtgt gtgtgtgtgt gtgtgtgtgt gtgtgtgtgt      60

gtgagagaga gagagagaga gagagacaga gagacagaga gagagagagc gc              112


<210>  45
<211>  106
<212>  DNA
<213>  Homo sapiens

<400>  45
gagtgtgtgt gtgtgtgtgt gtgtgtgtgt gtgtgtgtgt gtgtgtgaga gagagagaga      60

gagagagaga gagagaggaa gagagagaca gagagaggga gcgcgc                      106


<210>  46
<211>  90
<212>  DNA
<213>  Homo sapiens

<400>  46
gagtgtgtgt gtgtgtgtgt gtgtgtgtgt gtgtgtgtga gagagagaga gagagagaga      60

ggaagagaga gacagagaga gggagcgcgc                                        90


<210>  47
<211>  128
<212>  DNA
<213>  Homo sapiens

<400>  47
gagtgtgtgt gtgtgtgtgt gtgtgtgtgt gtgtgtgtgt gtgtgtgtgt gtgtgtgtga      60

gagagagaga gagagagaga gagagagaga gagagagagg aagagagaga cagagagagg      120

gagcgcgc                                                                128


<210>  48
<211>  110
<212>  DNA
<213>  Homo sapiens

<400>  48
gagtgtgtgt gtgtgtgtgt gtgtgtgtgt gtgtgtgtgt gtgtgtgtga gagagagaga      60

gagagagaga gagagagaga ggaagagaga gacagagaga gggagcgcgc                  110


<210>  49
<211>  76

<212>  DNA
<213>  Homo sapiens

<400>  49
gagagagtgt gtgtgtgtgt gtgtgtgtgt gtgagagaga gagagagaga ggaagagaca      60

gagagaggga gcgcgc                                                      76


<210>  50
<211>  80
<212>  DNA
<213>  Homo sapiens

<400>  50
gagagagtgt gtgtgtgtgt gtgtgtgtgt gtgtgagaga gagagagaga gagaggaaga      60

gacagagaga gggagcgcgc                                                  80


<210>  51
<211>  84
<212>  DNA
<213>  Homo sapiens

<400>  51
gagtgtgtgt gtgtgtatgt gtgtatgtgt gtgtgtgaga gagagagaca gagaggaaga      60

gagagacaga gagagggagc gcgc                                             84


<210>  52
<211>  64
<212>  DNA
<213>  Homo sapiens

<400>  52
gtatgtgtgt gtgtgtgtgt gtgagagaga gagagagaga cagaggaaga gagagagagc      60

gtgc                                                                   64


<210>  53
<211>  86
<212>  DNA
<213>  Homo sapiens

<400>  53
gtatgtgtgt gtgtgtgtgt gtgtgtgaga gagagagaga gagagagaga gagacagaca      60

gaggaagaga gagagagcgt gtgcgc                                           86


<210>  54
<211>  36
<212>  DNA
<213>  Homo sapiens

<400>  54
gtctgtgtgt gtgtgtgaga gagagagaga gagaga                                36


<210>  55
<211>  48
<212>  DNA
<213>  Homo sapiens

<400>  55

gtgtgtgtgt gtgtgtgtgt gtgtgtgaga gagagagaga gagagaga 48

<210> 56
<211> 76
<212> DNA
<213> Homo sapiens

<400> 56
gtgtgtgtgt gtgtgtgtgt gtgtgtgtgt gtgtgtgtgt gtgtgtgtga gagagagaga 60

gagagagaga gagaga 76

<210> 57
<211> 42
<212> DNA
<213> Homo sapiens

<400> 57
gtgtgtgtgt gtgtgtgtgt gtgtgtgtgt gagagagaga ga 42

<210> 58
<211> 35
<212> DNA
<213> Homo sapiens

<400> 58
gtgtttgtgt gttgagagag agacacacac acaca 35

<210> 59
<211> 35
<212> DNA
<213> Homo sapiens

<400> 59
gtgtttgtgt gttgagagag agagacacac acaca 35

<210> 60
<211> 94
<212> DNA
<213> Macaca mulatta

<400> 60
gtgtgtgtct gtgtgtgtgt gtgtgtgtgt gtgtgagaga gagagagaga gagagagaga 60

gagagagaga gaggaagaca gagagcgcgc gcgc 94

<210> 61
<211> 96
<212> DNA
<213> Macaca mulatta

<400> 61
gtgtgtgtct gtgtgtgtgt gtgtgtgtgt gtgtgagaga gagagagaga gagagagaga 60

gagagagaga gagaggaaga cagagagcgc gcgcgc 96

<210> 62
<211> 72
<212> DNA
<213> Macaca mulatta

<400>  62
gtgtgtgtgt gtgtgtgtgt gtgtgtgtgt gtgtgtgtgt gtgaaagaga gagagagcga      60

gcgagcgaga gc                                                          72


<210>  63
<211>  84
<212>  DNA
<213>  Macaca mulatta

<400>  63
gtgtgtgtgt gtgtgtgtgt gtgtgtgtgt gtgtgtgtgt gtgtgtgtgt gtgtgaaaga      60

gagagagagc gagcgagcga gagc                                             84


<210>  64
<211>  74
<212>  DNA
<213>  Macaca mulatta

<400>  64
gtgtgtgtgt gtgtgtgtgt gtgtgtgtgt gtgtgtgtga gacagagaga gagagaggga      60

gagagagagc gcgc                                                        74


<210>  65
<211>  76
<212>  DNA
<213>  Macaca mulatta

<400>  65
gtgtgtgtgt gtgtgtgtgt gtgtgtgtgt gtgtgtgtgt gtgacagaga gagagagagg      60

gagagagaga gagcgc                                                      76


<210>  66
<211>  60
<212>  DNA
<213>  Macaca mulatta

<400>  66
gtgtgtgtct gtgtgtgtgt gtgtgtgtgt gtgagagaga gagagagaga gagagagagc      60


<210>  67
<211>  40
<212>  DNA
<213>  Macaca mulatta

<400>  67
gtgtgagaaa gaaagagaaa gaaagagaga gagcgcgcgc                            40


<210>  68
<211>  44
<212>  DNA
<213>  Macaca mulatta

<400>  68
gtgtgagaaa aagagaaaga gaaagaaaga gagagagtgc gcgc                       44


<210>  69
<211>  22

<212>  DNA
<213>  Macaca mulatta

<400>  69
gtgtaagaaa gagagcgtgc gc                                              22


<210>  70
<211>  40
<212>  DNA
<213>  Macaca mulatta

<400>  70
gtgtgagaaa gagagagaga gaggaagaga gagcgcgcgc                           40


<210>  71
<211>  132
<212>  DNA
<213>  Macaca mulatta

<400>  71
gtgtgtctgt gagtgtgtgt gtgtgtgtgt gtgtgtgtgt gtgagagaga gtgtgtgtgt      60

gtgtgtgtgt gtgagagaga gagacagaga cagagagaca gagagagaga gacagagaca     120

gagagagcgc gc                                                         132


<210>  72
<211>  134
<212>  DNA
<213>  Macaca mulatta

<400>  72
gtgtgtctgt gagtgtgtgt gtgtgtgtgt gtgtgtgtgt gtgtgagaga gagtgtgtgt      60

gtgtgtgtgt gtgtgagaga gagagacaga gacagagaga cagagagaga gagacagaga     120

cagagagagc gcgc                                                       134


<210>  73
<211>  70
<212>  DNA
<213>  Macaca mulatta

<400>  73
gtgtgtgtgt gtgtgtgtga gagtgtgtgt ggggggggga gggggagaga gagagggaga      60

gagcgcgcgc                                                             70


<210>  74
<211>  84
<212>  DNA
<213>  Macaca mulatta

<400>  74
gtgtgtgtgt gtgtgtgtgt gtgtgtgtgt gtgtgtgtga gagagagaga gagagagaga      60

gagagagaga gagagagaga gcgc                                             84


<210>  75
<211>  80
<212>  DNA
<213>  Macaca mulatta

<400> 75
gtgtgtgtgt gtgtgtgtgt gtgtgtgtgt gtgtgtgtgt gtgtgagaga gagagagaga 60

gagagagaga gagagagcgc 80


<210> 76
<211> 64
<212> DNA
<213> Macaca mulatta

<400> 76
gtgtgtgtgt aagagtgtgt gtgtgtgtgt gtgtgtgaga gagagagaga gagagagaga 60

gagc 64


<210> 77
<211> 74
<212> DNA
<213> Macaca mulatta

<400> 77
gtgtgtgtgt gagtgtgtgt cagagagaga gagagagaga cagagacaga gagagagaca 60

gagacagagc gcgc 74


<210> 78
<211> 108
<212> DNA
<213> Macaca mulatta

<400> 78
gtgtgtgtgt gagtgtgtgt gagtgtgtgt gagtgtgtgt gtgtgtgtgt gtgtgtcaga 60

gagagagaga gagacagaga cagagagaga gacagagaca gagcgcgc 108


<210> 79
<211> 152
<212> DNA
<213> Macaca mulatta

<400> 79
gtgtgtgtgt gagtgtgtgt gtgtgtgtgt gagagtgtgt gtctgtgtgt gtgtgtgagt 60

gtgagtgtgt gtatgtgaga gagagagaga gagagagaca gagacagaga cagagacaga 120

gacagagaca gagacagaga gagagcgcgc gc 152


<210> 80
<211> 146
<212> DNA
<213> Macaca mulatta

<400> 80
gtgtgtgtgt gagtgtgtgt gtgtgagagt gtgtgtctgt gtgtgtgtgt gagtgtgagt 60

gtgtgtatgt gagagagaga gagagagaga gagagagaga gagagagaga gagagacaga 120

gacagagaca gagagagagc gcgcgc 146


<210> 81
<211> 62

<212>  DNA
<213>  Macaca mulatta

<400>  81
gtgtgtgtgt gtgtgtgtgt gtgtgtgaga gagagagaga gagagagaga gagagagaga      60

gc                                                                    62


<210>  82
<211>  80
<212>  DNA
<213>  Macaca mulatta

<400>  82
gtgtgtgtgt gtgtgtgtgt gtgagtgtgt gtgtgagaga gagagagaga gagagagaga      60

gagagagaga gagagagagc                                                 80


<210>  83
<211>  80
<212>  DNA
<213>  Macaca mulatta

<400>  83
gtgtgtgtgt gtgtgtgtgt gtgtgtgtgt gtgtgtgtgt gagagagaga gagagagaga      60

gagagagaga gagagagagc                                                 80


<210>  84
<211>  102
<212>  DNA
<213>  Macaca mulatta

<400>  84
gtgtgtgtgt gtgtgtgtgt gtgtgtgtgt gtgtgtgtgt gtgagagaga gagagagaga      60

gagagagaga gagagagaga gagagagaga gagagagaga gc                       102


<210>  85
<211>  68
<212>  DNA
<213>  Macaca mulatta

<400>  85
gtgtgtgtgt gtgtgtgtgt gtgtgtgtgt gtgtgtgaga gagagagaga gagagagaga      60

gagagagc                                                              68


<210>  86
<211>  84
<212>  DNA
<213>  Macaca mulatta

<400>  86
gtgtgtgtgt gtgtgtgtgt gtgtgtgtgt gtgtgtgtga gagagagaga gagagagaga      60

gagagagaga gagagagaga gagc                                            84


<210>  87
<211>  68
<212>  DNA
<213>  Macaca mulatta

<400>  87
gtgtgtgtgt gtgtgtgtgt gtgtgtgtga gagagagaga gagagagaga gagagagaga      60

gagagcgc                                                                68


<210>  88
<211>  98
<212>  DNA
<213>  Macaca mulatta

<400>  88
gtgtgtgtgt gtgtgtgtgt gtgtgtgtgt gtgtgtgtgt gagagagaga gagagacaga      60

gagacagaga gagagagaca gagacagaga gagcgcgc                              98


<210>  89
<211>  98
<212>  DNA
<213>  Macaca mulatta

<400>  89
gtgtgtgtgt gtgtgtgtgt gtgtgtgtgt gtgtgtgtgt gtgagagaga gagagagaga      60

gagagagaca gagagacaga cagagagaga gagagcgc                              98


<210>  90
<211>  90
<212>  DNA
<213>  Macaca mulatta

<400>  90
gtgtgtgtgt gtgtgtgtgt gtgtgtgtgt gtgtgagaga gagagagaga gagagagaga      60

cagagagaca gacagagaga gagagagcgc                                        90


<210>  91
<211>  106
<212>  DNA
<213>  Macaca mulatta

<400>  91
gtgtgtgtgt gtgtgtgtgt gtgtgtgtgt gtgtgtgtgt gtgagagaga gagagagaga      60

gagagagaga gacagagaga gagacagaga cagagagagc gcgcgt                     106


<210>  92
<211>  64
<212>  DNA
<213>  Macaca mulatta

<400>  92
gagagagaga gagagagaga gagagagaga gagagagaga gagagagaga gagagagaga      60

gcgc                                                                    64


<210>  93
<211>  38
<212>  DNA
<213>  Macaca mulatta

<400>  93

gagtgtgtgt gtgtgtgtgt gtgagagaga gagagcgc 38

<210> 94
<211> 64
<212> DNA
<213> Macaca mulatta

<400> 94
gagtgtgtgt gtgtgtctgt gtgtgtgtgt gtttgtgtgt gtgtatgtga gagagagaga 60

gagc 64

<210> 95
<211> 40
<212> DNA
<213> Macaca mulatta

<400> 95
gagtatgtgt gtgtgtgtga gagagagaga gagagagcgc 40

<210> 96
<211> 42
<212> DNA
<213> Macaca mulatta

<400> 96
gagtatgtgt gtgtgtgtga gagagagaga gagagagagc gc 42

<210> 97
<211> 78
<212> DNA
<213> Macaca mulatta

<400> 97
gagtgtgtgt gtgtgtctgt gtgtgtgtgt atgtgagaga gagagagaga gagagagaga 60

gagagagaga aagcgcgc 78

<210> 98
<211> 52
<212> DNA
<213> Macaca mulatta

<400> 98
gagtgtgtgt gtgtctgtgt gtgtgtgtat gtgagagaga gagagagagc gc 52

<210> 99
<211> 56
<212> DNA
<213> Macaca mulatta

<400> 99
gagtgtgtgt gtgtgtatct gtgtgtgtgt gtatgtgaga gagagagaga gagcgc 56

<210> 100
<211> 90
<212> DNA
<213> Macaca mulatta

<400> 100
gagtgtgtgt gtgtgtctgt gtgtgtgtgt gtgtgtgtga gagagagaga gagagagaga 60

gagagagaga gagagaggga gagagagcgc                                    90

<210>  101
<211>  80
<212>  DNA
<213>  Macaca mulatta

<400>  101
gagtgtgtgt gtgtgtctgt gtgtgtgtgt gtgtgtgtgt gtgagagaga gagagagaga      60

gagagaggga gagagagcgc                                                  80

<210>  102
<211>  66
<212>  DNA
<213>  Macaca mulatta

<400>  102
gtgtgtgtgt gtgtgtgtgt gtgtgtgtgt gtgtgtgtgt gtgtgtgtgt gtgtgtgaga      60

gagagc                                                                 66

<210>  103
<211>  84
<212>  DNA
<213>  Macaca mulatta

<400>  103
gtgtgtgtgt gtgtgtgtgt gtgtgtgaga gagagagaga gagacagaga gacagagaga      60

gagagagaga cagagagaga gagc                                             84

<210>  104
<211>  86
<212>  DNA
<213>  Macaca mulatta

<400>  104
gtgtgtgtgt gtgtgtgtgt gtgtgtgtgt gtgtgtgtgt gtgtgagaga gagagagaga      60

gagagagaga gagagagaga gagagc                                           86

<210>  105
<211>  54
<212>  DNA
<213>  Macaca mulatta

<400>  105
gtgtgtgtgt gtgtgtgtgt gtgtgtgtgt gtgtgagaga gagagagaga gagc            54

<210>  106
<211>  80
<212>  DNA
<213>  Macaca mulatta

<400>  106
gtgtgtgtgt gtgtgtgtgt gtgtgtgtgt gtgtgtgaga gagagagaga gagagagaga      60

gagagagaga gagagagagc                                                  80

<210> 107
<211> 92
<212> DNA
<213> Macaca mulatta

<400> 107
gagtgtgtgt gtgtgtctgt gtgtgtgtgt gtgtgtgtgt gtgtgtgaga gagagagaga 60

gagagagaga gagagagaga gagagaaaga gc 92

<210> 108
<211> 48
<212> DNA
<213> Macaca mulatta

<400> 108
gtgtgtgtgt gtgtgtgtgt gtgtgtgtgt atgagagaga gagagcgc 48

<210> 109
<211> 50
<212> DNA
<213> Macaca mulatta

<400> 109
gtgtgtgtgt gtgtgtgtgt gtgtgtgtgt gtatgagaga gagagagcgc 50

<210> 110
<211> 88
<212> DNA
<213> Macaca mulatta

<400> 110
gtgtgtgtgt gtgtgtgtgt gtgtgtgtgt gtgtgtgtgt gtgtgagaga gagagagaga 60

gagagagaga gagagagaga gagagagc 88

<210> 111
<211> 46
<212> DNA
<213> Macaca mulatta

<400> 111
gtgtgtgtgt gtgtgtgtgt gagagagaga gagagagaga gagagc 46

<210> 112
<211> 62
<212> DNA
<213> Macaca mulatta

<400> 112
gtgtgtgtgt gtgtgtgtgt gtgtgtgtgt gtgagagaga gagagagaga gagagagaga 60

gc 62

<210> 113
<211> 60
<212> DNA
<213> Macaca mulatta

<400> 113
gtgtgtgtgt gtgtgtgtgt gtgtgtgtgt gagagagaga gagagagaga gagagagcgc 60

<210>  114
<211>  170
<212>  DNA
<213>  Macaca mulatta

<400>  114
gtgtgtgtct gtgtgtctgt gtgtgtgtgt gtgtgtgtgt ctgtgtgtgt gtgtctgtgt      60

gtgtctgtgt ctgtgtgtgt gtgtctgtgt gtgtgtgtgt gtctgtgtgt gtgtgtctgt     120

gtgtgtgtgt gtgtgtgaga gagagagaga gagagagaga gagagagcgc                170


<210>  115
<211>  88
<212>  DNA
<213>  Macaca mulatta

<400>  115
gagagtgtgt gtgtgtgtgt gtgtgtgtga gagagagaga cagagagaga gaaagagaga      60

gagagagaga cagagacaga gagcgcgc                                          88


<210>  116
<211>  58
<212>  DNA
<213>  Macaca mulatta

<400>  116
gtgtgtgtgt gtgtgtgtgt gagagagaga gagagagaga gagagagaga gagagagc        58


<210>  117
<211>  84
<212>  DNA
<213>  Macaca mulatta

<400>  117
gtgtgtgtgt gtgtgtgtgt gtgtgtgtgt gtgtgtgtgt gagagagaga gagagagaca      60

gacagagaca gagagagaga gcgc                                              84


<210>  118
<211>  86
<212>  DNA
<213>  Macaca mulatta

<400>  118
gtgtgtgtgt gtgtgtgtgt gtgtgtgtgt gtgtgtgtgt gtgagagaga gagagagaga      60

cagacagaga cagagagaga gagcgc                                            86


<210>  119
<211>  68
<212>  DNA
<213>  Macaca mulatta

<400>  119
gtgtgtgtgt gtgtgtgtgt gtgtgagtgt gtgtgtgtga gacagagaga gagagagaga      60

gagagcgt                                                                68


<210>  120

<211>  82
<212>  DNA
<213>  Macaca mulatta

<400>  120
gtgtgtgtct gtgtgtgtgc atgtgtgtgt gtgtgtgaga gagagagaga gagagagaca      60

gagagagaga gagagagagc gc                                                82


<210>  121
<211>  94
<212>  DNA
<213>  Macaca mulatta

<400>  121
gtgtgtgtgt gtgtgtgtgt gtgtgtgtgt gtgtgagaga gagagagaga aagagagaca      60

gagagagaga cagagagaga gacagagcgc gcgc                                   94


<210>  122
<211>  98
<212>  DNA
<213>  Macaca mulatta

<400>  122
gtgtgtgtgt gtgtgtgtgt gtgtgtgtgt gtgtgtgaga gagagagaga gagaaagaga      60

gacagagaga gagacagaga gagagacaga gcgcgcgc                               98


<210>  123
<211>  54
<212>  DNA
<213>  Macaca mulatta

<400>  123
gtgtgtgtgt gtgtgtgtgt gtgtgagaga gagagagaga gagagagcga gcgc             54


<210>  124
<211>  112
<212>  DNA
<213>  Macaca mulatta

<400>  124
gtgtgtgtgt gtgtgcgtgt gtgtgtgtgt gtgtgtgtgt gtgtgtgtgt gagagagaga      60

gagagacaga gacagagaga cagagagaga gacagagaca gagagagcgc gc              112


<210>  125
<211>  92
<212>  DNA
<213>  Macaca mulatta

<400>  125
gtgtgtgtgt gtgtgtgtgt gtgtgtgtgt gtgtgtgtga gagagagaga gagagagaga      60

gagagacaga gacagagaca gagacagaga gc                                     92


<210>  126
<211>  112
<212>  DNA
<213>  Macaca mulatta

<400>  126
gtgtgtgtgt gtgtgtgtgt gtgtgtgtgt gtgtgtgtgt gtgtgtgtgt gagagagaga      60

gagagagaga gagagagaga gagagacaga gacagagaca gagacagaga gc             112


<210>  127
<211>  78
<212>  DNA
<213>  Macaca mulatta

<400>  127
gagtgtgtgt gtgtgtgtgt gtgtgtgtgt gtgtgtgtgt gagagagaga gagagagaga      60

gagagagaga gagagcgc                                                    78


<210>  128
<211>  94
<212>  DNA
<213>  Macaca mulatta

<400>  128
gagtgtgtgt gtgtgtgtgt gtgtgtgtgt gtgtgtgtgt gtgtgagaga gagagagaga      60

gagagagaga gagagagaga gagagagaga gcgc                                  94


<210>  129
<211>  70
<212>  DNA
<213>  Macaca mulatta

<400>  129
gtgtgtgtgt gtgtgtgtgt gtgtgtgtgt gtgtgtgtgt gtcagagaga gagagagaga      60

gagagagcgc                                                             70


<210>  130
<211>  66
<212>  DNA
<213>  Macaca mulatta

<400>  130
gtgtgtgtgt gagtgtgtgt gtgtgagtgt gtgtgtgagt gtgtgtgtgt gagagagaga      60

gagagc                                                                 66


<210>  131
<211>  70
<212>  DNA
<213>  Macaca mulatta

<400>  131
gtgtgtgtgt gtgtgagtgt gtgtgtgtga gtgtgtgtgt gagtgtgtgt gtgagagaga      60

gagagagagc                                                             70


<210>  132
<211>  100
<212>  DNA
<213>  Macaca mulatta

<400>  132
gtgtgtgtgt gagtgtgtgt gtgtgtgtgt gtgtgtgtgt ctgagagaga gacagagaca      60

gagagacaga gagagagaca gagacagaga gagcccgcgc                              100

<210>  133
<211>  96
<212>  DNA
<213>  Macaca mulatta

<400>  133
gtgtgtgtgt gagtgtgtgt gagtgtgtgt gtgtgtgaga gagagagaca gagacagaga      60

gacagagaga gagacagaga cagagagagc ccgcgc                                96